# EUROPEAN PATENT APPLICATION

(11) **EP 0 732 378 A2**
(43) Date of publication of application: **18.09.1996**
(21) Application number: 96301665.4
(22) Date of filing: 12.03.1996
(51) Int. Cl.: C09B 61/00, C09B 67/54, C07B 63/00, C07C 403/00

(54) **Method for purifying xanthophyll compound**

(30) Priority: 16.03.1995 JP 57566/95
(71) Applicant: NIPPON OIL CO. LTD., Minato-ku Tokyo (JP)
(72) Inventor: Kitaoka, Mitomitsu, Yokohama, Kanagawa (JP); Tsubokura, Akira, Yokohama, Kanagawa (JP); Kiyota, Takashi, Yokohama, Kanagawa (JP)
(74) Representative: Hallybone, Huw George

(57) **Abstract**

A method for purifying a xanthophyll compound involves the steps of: (a) adding a nonpolar solvent and water to an aqueous organic solvent solution containing the xanthophyll compound; (b) subjecting to liquid-liquid extraction to thereby obtain a nonpolar solvent phase; and (c) subjecting the nonpolar solvent phase to at least one step selected from the following steps (c-1) to (c-3): step (c-1) of concentrating the nonpolar solvent phase; step (c-2) of adding water to the nonpolar solvent phase followed by further subjecting at least once to liquid-liquid extraction; and step (c-3) of further adding a nonpolar solvent to the nonpolar solvent phase whereby the xanthophyll compound is precipitated.

## Description

### BACKGROUND OF THE INVENTION

This invention relates to a method for purifying a xanthophyll compound in which impurities other than the xanthophyll compound are removed from an aqueous organic solvent solution containing the xanthophyll compound for purifying the xanthophyll compound to high purity.

The xanthophyll compound is a generic name of carotenoid compounds containing oxygen atoms in the molecule thereof, and is a red-hued or yellow-hued pigment widely distributed in microorganisms or algae, plant or animal tissues or organs. Recently, the usage for the xanthophyll compound is increasing in the field of food additives as colorants for food or beverage or as colorants for fish meat such as salmon or trout, skin parts of the fish or eggs of fowl, such as hens. In addition, the xanthophyll compound is known to have an antioxidation action and its usage as an antioxidant is promising. It has also been found that certain xanthophyll compounds exhibit cancer-prohibiting properties such that they may be expected in future to be employed as pharmaceuticals.

Of the xanthophyll compounds, astaxanthin, canthaxanthin and zeaxanthin are produced industrially by a synthetic method (Pure and Applied Chemistry, 63(1), 35-44 (1991)) and are employed as feed additives for coloration. Recently, use of a synthesized product as food or feed additive is becoming more and more difficult in consideration of safety. Thus, in conjunction with the general preference for natural products, a strong demand has been raised for technology of producing natural carotenoid compounds in substitution for synthesized products.

A number of reports have been made in connection with the technique of extracting xanthophyll compounds from natural products. For example, the method of extracting the compounds with aqueous organic solvents, such as acetone, tetrahydrofuran, dioxane, pyridine or cyclohexanone, is thought to be effective. However, if the xanthophyll compounds are extracted from microorganisms, algae or the tissues or organs of plants or animals using these solvents, polar lipid, neutral lipid or carotenoid compounds other than the xanthophyll compounds are co-extracted as impurities. Although these impurities may be removed by chromatography, this method is economically disadvantageous since a large quantity of solvents needs to be employed. Thus, an economically meritorious method capable of removing these impurities efficiently has been desired.

### SUMMARY OF THE INVENTION

It is therefore an object of the present invention to provide a method for purifying a xanthophyll compound whereby the xanthophyll compound may be purified and isolated easily and efficiently by removing impurities from natural products.

The above and other objects of the invention will become apparent from the following description.

According to the present invention, there is provided a method for purifying a xanthophyll compound comprising the steps of (a) adding a nonpolar solvent and water to an aqueous organic solvent solution containing the xanthophyll compound; (b) subjecting to liquid-liquid extraction to thereby obtain a nonpolar solvent phase; and (c) subjecting the nonpolar solvent phase to at least one step selected from the group consisting of the following steps (c-1) to (c-3): step (c-1) of concentrating the nonpolar solvent phase; step (c-2) of adding water to the nonpolar solvent phase followed by further subjecting at least once to liquid-liquid extraction; and step (c-3) of further adding a nonpolar solvent to the nonpolar solvent phase whereby the xanthophyll compound is precipitated.

### PREFERRED EMBODIMENTS OF THE INVENTION

The present invention will be explained in further detail hereinbelow.

In the purifying method of the present invention, an aqueous organic solvent solution containing a xanthophyll compound is employed as a starting material in step (a). Although there is no limitation to the aqueous organic solvent solution containing the xanthophyll compound, it may preferably be produced by dissolving a material containing the xanthophyll compound in an aqueous organic solvent, or by extracting a material already extracted and containing the xanthophyll compound with an aqueous organic solvent. More preferably, it may be produced by extracting a natural material containing the xanthophyll compound, such as microorganisms, algae or the tissues or organs of plants or animals, with an aqueous organic solvent.

Although there is no particular limitation to the aqueous organic solvent, it may preferably be enumerated by acetone, tetrahydrofuran, dioxane, pyridine, cyclohexanone or mixtures thereof thought to be effective for extracting the xanthophyll compounds.

In the purifying method of the present invention, a nonpolar solvent and water are added to the aqueous organic solvent solution containing the xanthophyll compound in step (a) and liquid-liquid extraction is carried out in step (b) to obtain a nonpolar solvent phase.

Although there is no limitation to the nonpolar solvent, it may preferably be enumerated by hexane, heptane, octane, cyclohexane, benzene, toluene, xylene, petroleum ether, naphtha, kerosene and n-paraffin. Most preferred of these nonpolar solvents are hexane, cyclohexane and petroleum ether. These solvents may be used alone or in combination.

The water is not limited to pure water and preferably may be enumerated by distilled water, ion-exchanged water, city water, industrial water, aqueous solutions of salts, such as an aqueous sodium hydroxide solution or an aqueous sodium chloride solution, e.g. brine in which table salt, sodium chloride or sodium hydroxide is added to the aforementioned water, and sea water.

Although there is no particular limitation to the amounts of the nonpolar solvent and water added to the aqueous organic solvent solution containing the xanthophyll compound, the amount of the nonpolar solvent is preferably 0.1 to 20 parts by volume and more preferably 0.2 to 10 parts by volume to 1 part by volume of the aqueous organic solvent solution containing the xanthophyll compound, while the amount of water is usually not less than 0.1 part by volume, e.g. 0.1 to 30 parts by weight and preferably 0.2 to 10 parts by volume to 1 part by volume of the aqueous organic solvent solution containing the xanthophyll compound.

There is no particular limitation to the liquid-liquid extraction in step (b) provided the nonpolar solvent phase may thereby be obtained. Thus, it is possible to use any desired method for liquid-liquid extraction. For example, the nonpolar solvent phase may be produced by a batch type method for liquid-liquid extraction consisting of agitating a liquid mixture composed of the aqueous organic solvent solution containing the xanthophyll compound, the nonpolar solvent and the water in an agitating vessel, or by continuous liquid-liquid extraction using a mixer-settler extractor. There is no particular limitation to the extraction temperature, which may preferably be 0°C to 100°C and more preferably 10°C to 80°C, while there is also no limitation to the liquid-liquid extraction time duration, which may preferably be 10 seconds to 10 hours.

With the purifying method of the present invention, at least one of the step (c-1) of concentrating the nonpolar solvent phase; step (c-2) of adding water to the nonpolar solvent phase followed by further subjecting at least once to liquid-liquid extraction; and step (c-3) of further adding a nonpolar solvent to the nonpolar solvent phase, is carried out in the step (c) for precipitating the xanthophyll compound.

The above step (c-1) may be carried out by heating at atmospheric pressure or reduced pressure using a rotary evaporator or the like for distilling off part or all of the solvent in the nonpolar solvent phase. Although there is no particular limitation to the factor of concentration, it is preferably 1.5 to 1,000 folds. By this step (c-1), the nonpolar solvent phase is concentrated for selectively precipitating only the xanthophyll compound.

The above step (c-2) may be carried out by a method consisting of adding water to the nonpolar solvent phase and carrying out liquid-liquid extraction in accordance with a batch system as in the case of the above-mentioned liquid-liquid extraction. Alternatively, the step (c-2) may be carried out by continuous liquid-liquid extraction using a mixer-settler extractor. The water added is not limited to pure water and the aqueous solutions as well as the water enumerated for the step (a) may also be employed. There is no limitation to the amount of water added for the step (c-2) and usually may be not less than 0.1 part by volume and, for example 0.1 to 30 parts by volume and preferably 0.2 to 10 parts by volume to 1 part by volume of the nonpolar solvent phase. There is also no limitation on the temperature for liquid-liquid extraction which may preferably be 0°C to 100°C and more preferably 10°C to 80°C. Although there is also no particular limitation to the time of liquid-liquid extraction, it is preferably 10 seconds to 10 hours. The liquid-liquid extraction with water may be repeated plural times. By lowering the aqueous organic solvent concentration in step (c-2), only the xanthophyll compound may be precipitated selectively.

In the step (c-3), the xanthophyll compound is precipitated by simply adding a nonpolar solvent to the nonpolar solvent phase. The nonpolar solvent enumerated above for the step (a) may be used as the nonpolar solvent for the step (c-3). That is, the same or different nonpolar solvent may be used for the step (c-3). Although no limitations are imposed on the amount of the nonpolar solvent added, it is usually not less than 0.1 part by volume, typically 0.1 to 30 parts by volume and preferably 0.2 to 10 parts by volume to one part by volume of the nonpolar solvent phase.

Although the xanthophyll compound may be precipitated efficiently and with high purity by carrying out one of the steps (c-1) to (c-3) alone, these steps may be suitably combined with one another, if so desired.

The xanthophyll compound precipitated by at least one of the steps (c-1) to (c-3) may be recovered by any suitable method. There is no particular limitation to the recovering method which may usually be carried out by filtration or centrifugal separation.

With the purifying method according to the present invention, carotenoid compounds other than the xanthophyll compound may be removed from the aqueous organic solvent solution containing the xanthophyll compound in addition to the impurities other than the carotenoid compounds. It should be noted that the carotenoid compounds other than the xanthophyll compound were heretofore difficult to separate and remove from the aqueous organic solvent solution containing the xanthophyll compound.

There is no particular limitation to the xanthophyll compound that may be purified in accordance with the purifying method of the present invention, provided that the compound is a carotenoid compound containing an oxygen atom in the molecules thereof. Examples of the xanthophyll compound includes astaxanthin, canthaxanthin, zeaxanthin, adonixanthin, adonirubin, β-cryptoxanthin, echinenone, asteroidenone, 3-hydroxyechinenone, rhodoxanthin, fucoxanthin, lutein, capsanthin and capsorubin.

With the purifying method of the present invention,. the xanthophyll compound, which cannot be purified with the conventional technique, can be purified easily and efficiently. In particular, the purifying method of the present invention may be usefully applied to purification from a material derived from natural products. The purified xanthophyll compounds may be applied to a variety of usages, including food additives, feed additives, starting materials therefor, or starting materials for pharmaceuticals.

### EXAMPLES OF THE INVENTION

The present invention will be explained with reference to Preparation Examples and Examples, which are merely illustrative and are not intended for limiting the scope of the invention.

An E-396 strain employed in the Preparation Examples 1 and 2, which was identified as not belonging to any known genus, was deposited at the NATIONAL INSTITUTE OF BIOSCIENCE AND HUMAN TECHNOLOGY, AGENCY OF INDUSTRIAL SCIENCE AND TECHNOLOGY on April 27, 1993 and has been accorded accession number FERM BP-4283. FERM BP-4283 has been accepted for deposit under the Budapest Treaty on the International Recognition of the Deposit of Microorganisms for the Purposes of Patent Procedure. All restrictions on the availability to the public of FERM BP-4283 will be irrevocably removed upon the granting of a patent.

### Preparation Example 1

### Preparation of Acetone-Extracted Colored Liquid

To 100 g (wet weight) of an E-396 cultured strain (FERM BP-4283), which was bacteria producing astaxanthin, adonixanthin, adonirubin, canthaxanthin, and echinenone, was added 1 liter of acetone. The resulting mass was agitated for one hour and filtered to produce an acetone-extracted colored liquid (A). The xanthophyll compound concentration in the colored liquid (A) was 80 ppm. The purity of the xanthophyll compound in the solid content contained in the colored liquid (A) was 9.7%. The colored liquid (A) contained β-carotene, phospholipid and neutral lipid as impurities.

### Preparation Example 2

### Preparation of Tetrahydrofuran (THF)-Extracted Colored Liquid

To 250 g (wet weight) of an E-396 cultured strain (FERM BP-4283), which was bacteria producing astaxanthin, adonixanthin, adonirubin, canthaxanthin, and echinenone, was added 1 liter of tetrahydrofuran (THF). The resulting mass was agitated for one hour and filtered to produce a THF-extracted colored liquid (B). The xanthophyll-compound concentration in the colored liquid (B) was 350 ppm. The purity of the xanthophyll compound in the solid content contained in the colored liquid (B) was 11.2%. The colored liquid (B) contained β-carotene, phospholipid and neutral lipid as impurities.

### Example 1-1

To 200 ml of the acetone-extracted colored liquid (A) prepared in the Preparation Example 1,. were added 200 ml of hexane and 200 ml of a 1 wt% aqueous solution of sodium chloride and agitated for one hour. The resulting solution was allowed to stand for sampling 223 ml of a hexane phase as an upper phase. The produced hexane phase was concentrated under vacuum up to 20 ml using a rotary evaporator. The concentrated solution was allowed to stand at 4°C for 12 hours and the resulting precipitate was recovered by filtration. As a result, 11.9 mg of dark purplish powders were obtained. The purity of the xanthophyll compound in the powders, as confirmed by high performance liquid chromatography, was found to be 98%.

### Example 1-2

To 200 ml of the acetone-extracted colored liquid (A) prepared in the Preparation Example 1, were added 200 ml of hexane and 200 ml of a 1 wt% aqueous solution of sodium chloride and agitated for one hour. The resulting solution was allowed to stand for sampling 223 ml of a hexane phase as an upper phase. The produced hexane phase was concentrated under vacuum up to 100 ml using a rotary evaporator. The concentrated solution was allowed to stand at 4°C for 12 hours and the resulting precipitate was recovered by filtration. As a result, 10.8 mg of dark purplish powders were obtained. The purity of the xanthophyll compound in the powders, as confirmed by high performance liquid chromatography, was found to be 99%.

### Example 1-3

To 200 ml of the acetone-extracted colored liquid (A) prepared in the Preparation Example 1, were added 200 ml of hexane and 200 ml of city water and agitated for one hour. The resulting solution was allowed to stand for sampling 218 ml of an upper hexane phase. The hexane phase thus obtained was concentrated under vacuum to 20 ml using a rotary evaporator. The resulting concentrated liquid was allowed to stand at 4°C for 12 hours and the precipitate was recovered by filtration. As a result, 12.3 mg of dark purplish powders were obtained. The purity of the xanthophyll compound in the powders, as confirmed by high performance liquid chromatography, was found to be 98%.

### Example 1-4

To 200 ml of the acetone-extracted colored liquid (A) prepared in the Preparation Example 1, were added 200 ml of petroleum ether and 200 ml of a 1 wt% aqueous solution of sodium chloride and agitated for one hour. The resulting solution was allowed to stand for sampling 221 ml of a petroleum ether phase as an upper phase. The produced petroleum ether phase was concentrated under vacuum up to 20 ml using a rotary evaporator. The concentrated solution was allowed to stand at 4°C for 12 hours and the resulting precipitate was recovered by filtration. As a result, 11.9 mg of dark purplish powders were obtained. The purity of the xanthophyll compound in the powders, as confirmed by high performance liquid chromatography, was found to be 99%.

### Example 1-5

To 200 ml of the acetone-extracted colored liquid (A) prepared in the Preparation Example 1, were added 200 ml of cyclohexane and 200 ml of a 1 wt% aqueous solution of sodium chloride and agitated for one hour. The resulting solution was allowed to stand for sampling 226 ml of a cyclohexane phase as an upper phase. The produced cyclohexane phase was concentrated under vacuum up to 20 ml using a rotary evaporator. The concentrated solution was allowed to stand at 4°C for 12 hours and the resulting precipitate was recovered by filtration. As a result, 12.4 mg of dark purplish powders were obtained. The purity of the xanthophyll compound in the powders, as confirmed by high performance liquid chromatography, was found to be 97%.

### Example 1-6

To 200 ml of the acetone-extraoted colored liquid (A) prepared in the Preparation Example 1, were added 200 ml of hexane and 100 ml of a 1 wt% aqueous solution of sodium chloride and agitated for one hour. The resulting solution was allowed to stand for sampling 235 ml of a hexane phase as an upper phase. The produced hexane phase was concentrated under vacuum up to 20 ml using a rotary evaporator. The concentrated solution was allowed to stand at 4°C for 12 hours and the resulting precipitate was recovered by filtration. As a result, 12.6 mg of dark purplish powders were obtained. The purity of the xanthophyll compound in the powders, as confirmed by high performance liquid chromatography, was found to be 99%.

### Example 1-7

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. The produced hexane phase was concentrated under vacuum up to 10 ml using a rotary evaporator. The concentrated solution was allowed to stand at 4°C for 12 hours and the resulting precipitate was recovered by filtration. As a result, 55 mg of dark purplish powders were obtained. The purity *of* the xanthophyll compound in the powders, as confirmed by high performance liquid chromatography, was found to be 99%.

### Example 1-8

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. The produced hexane phase was concentrated under vacuum up to 20 ml using a rotary evaporator. The concentrated solution was allowed to stand at 4°C for 12 hours and the resulting precipitate was recovered by filtration. As a result, 52 mg of dark purplish powders were obtained. The purity of the xanthophyll compound in the powders, as confirmed by high performance liquid chromatography, was found to be 99%.

### Example 1-9

To 200 ml of the THF-extracted colored liquid (B) prepared in the Preparation Example 2, were added 200 ml of hexane and 200 ml of city water and agitated for one hour. The resulting solution was allowed to stand for sampling 301 ml of an upper hexane phase. The produced hexane phase was concentrated under vacuum up to 10 ml using a rotary evaporator. The concentrated liquid was allowed to stand at 4°C for 12 hours and the precipitate was recovered by filtration. As a result, 57 mg of dark purplish powders were obtained. The purity of the xanthophyll compound in the powders, as confirmed by high performance liquid chromatography, was found to be 100%.

### Example 1-10

To 200 ml of the THF-extracted colored liquid (B) prepared in the Preparation Example 2, were added 200 ml of petroleum ether and 200 ml of a 1 wt% aqueous solution of sodium chloride and agitated for one hour. The resulting solution was allowed to stand for sampling 316 ml of a petroleum ether phase as an upper phase. The produced petroleum ether phase was concentrated under vacuum up to 10 ml using a rotary evaporator. The concentrated solution was allowed to stand at 4°C for 12 hours and the resulting precipitate was recovered by filtration. As a result, 58 mg of dark purplish powders were obtained. The purity of the xanthophyll compound in the powders, as confirmed by high performance liquid chromatography, was found to be 98%.

### Example 1-11

To 200 ml of the THF-extracted colored liquid (B) prepared in the Preparation Example 2, were added 200 ml of cyclohexane and 200 ml of a 1 wt% aqueous solution of sodium chloride and agitated for one hour. The resulting solution was allowed to stand for sampling 305 ml of a cyclohexane phase as an upper phase. The produced cyclohexane phase was concentrated under vacuum up to 10 ml using a rotary evaporator. The concentrated solution was allowed to stand at 4°C for 12 hours and the resulting precipitate was recovered by filtration. As a result, 59 mg of dark purplish powders were obtained. The purity of the xanthophyll compound in the powders, as confirmed by high performance liquid chromatography, was found to be 99%.

### Example 1-12

To 200 ml of the THF-extracted colored liquid (B) prepared in the Preparation Example 2, were added 200 ml of hexane and 100 ml of a 1 wt% aqueous solution of sodium chloride and agitated for one hour. The resulting solution was allowed to stand for sampling 325 ml of a hexane phase as an upper phase. The produced hexane phase was concentrated under vacuum up to 10 ml using a rotary evaporator. The concentrated solution was allowed to stand at 4°C for 12 hours and the resulting precipitate was recovered by filtration. As a result, 52 mg of dark purplish powders were obtained. The purity of the xanthophyll compound in the powders, as confirmed by high performance liquid chromatography, was found to be 99%.

### Example 2-1

To 200 ml of the acetone-extracted colored liquid (A) prepared in the Preparation Example 1, were added 200 ml of hexane and 200 ml of a 1 wt% aqueous solution of sodium chloride and agitated for one hour. The resulting solution was allowed to stand for sampling 223 ml of a hexane phase as an upper phase. To the produced hexane phase were added 200 ml of a 1 wt% aqueous solution of sodium chloride and the resulting solution was agitated for one hour. The resulting solution was allowed to stand at 4°C for 12 hours and the resulting precipitate was recovered by filtration. As a result, 11.2 mg of dark purplish powders were obtained. The purity of the xanthophyll compound, as confirmed by high performance liquid chromatography, was found to be 99%.

### Example 2-2

To 200 ml of the acetone-extracted colored liquid (A) prepared in the Preparation Example 1, were added 200 ml of hexane and 200 ml of a 1 wt% aqueous solution of sodium chloride and agitated for one hour. The resulting solution was allowed to stand for sampling 223 ml of a hexane phase as an upper phase. To the produced hexane phase were added 400 ml of a 1 wt% aqueous solution of sodium chloride and the resulting solution was agitated for one hour. The resulting solution was allowed to stand at 4°C for 12 hours and the resulting precipitate was recovered by filtration. As a result, 11.8 mg of dark purplish powders were obtained. The purity of the xanthophyll compound in the powders, as confirmed by high performance liquid chromatography, was found to be 98%.

### Example 2-3

To 200 ml of the acetone-extracted colored liquid (A) prepared in the Preparation Example 1, were added 200 ml of hexane and 200 ml of city water and agitated for one hour. The resulting solution was allowed to stand for sampling 218 ml of a hexane phase as an upper phase. The resulting hexane phase was admixed with 200 ml of city water and the resulting solution was agitated for one hour. The resulting solution was allowed to stand at 4°C for 12 hours and the resulting precipitate was recovered by filtration. As a result, 11.4 mg of dark purplish powders were obtained. The purity of the xanthophyll compound in the powders, as confirmed by high performance liquid chromatography, was found to be 98%.

### Example 2-4

To 200 ml of the acetone-extracted colored liquid (A) prepared in the Preparation Example 1, were added 200 ml of petroleum ether and 200 ml of a 1 wt% aqueous solution of sodium chloride and agitated for one hour. The resulting solution was allowed to stand for sampling 221 ml of a petroleum ether phase as an upper phase. The resulting petroleum ether phase was admixed with 200 ml of city water and the resulting solution was agitated for one hour. The resulting solution was allowed to stand at 4°C for 12 hours and the resulting precipitate was recovered by filtration. As a result, 11.6 mg of dark purplish powders were obtained. The purity of the xanthophyll compound in the powders, as confirmed by high performance liquid chromatography, was found to be 99%.

### Example 2-5

To 200 ml of the acetone-extracted colored liquid (A) prepared in the Preparation Example 1, were added 200 ml of cyclohexane and 200 ml of a 1 wt% aqueous solution of sodium chloride and agitated for one hour. The resulting solution was allowed to stand for sampling 226 ml of a cyclohexane phase as an upper phase. The resulting cyclohexane phase was admixed with 200 ml of a 1 wt% aqueous solution of sodium chloride and the resulting solution was agitated for one hour. The resulting solution was allowed to stand at 4°C for 12 hours and the resulting precipitate was recovered by filtration. As a result, 11.9 mg of dark purplish powders were obtained. The purity of the xanthophyll compound in the powders, as confirmed by high performance liquid chromatography, was found to be 97%.

### Example 2-6

To 200 ml of the acetone-extracted colored liquid (A) prepared in the Preparation Example 1, were added 200 ml of hexane and 100 ml of a 1 wt% aqueous solution of sodium chloride and agitated for one hour. The resulting solution was allowed to stand for sampling 235 ml of a hexane phase as an upper phase. The resulting hexane phase was admixed with 200 ml of city water and the resulting solution was agitated for one hour. The resulting solution was allowed to stand at 4°C for 12 hours and the resulting precipitate was recovered by filtration. As a result, 11.5 mg of dark purplish powders were obtained. The purity of the xanthophyll compound in the powders, as confirmed by high performance liquid chromatography, was found to be 97%.

### Example 2-7

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. The resulting hexane phase was further liquid-liquid extracted thrice with 200 ml of a 1 wt% aqueous solution of sodium chloride. The resulting solution was allowed to stand at 4°C for 12 hours and the resulting precipitate was recovered by filtration. As a result, 55 mg of dark purplish powders were obtained. The purity of the xanthophyll compound in the powders, as confirmed by high performance liquid chromatography, was found to be 99%.

### Example 2-8

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. The resulting hexane phase was further liquid-liquid extracted thrice with 100 ml of a 1 wt% aqueous solution of sodium chloride. The resulting solution was allowed to stand at 4°C for 12 hours and the resulting precipitate was recovered by filtration. As a result, 50 mg of dark purplish powders were obtained. The purity of the xanthophyll compound in the powders, as confirmed by high performance liquid chromatography, was found to be 98%.

### Example 2-9

To 200 ml of the THF-extracted colored liquid (B) prepared in the Preparation Example 2, were added 200 ml of hexane and 200 ml of city water and agitated for one hour. The resulting solution was allowed to stand for sampling 301 ml of a hexane phase as an upper phase. The resulting hexane phase was further liquid-liquid extracted thrice with 200 ml of city water. The resulting solution was allowed to stand at 4°C for 12 hours and the resulting precipitate was recovered by filtration. As a result, 59 mg of dark purplish powders were obtained. The purity of the xanthophyll compound in the powders, as confirmed by high performance liquid chromatography, was found to be 99%.

### Example 2-10

To 200 ml of the THF-extracted colored liquid (B) prepared in the Preparation Example 2, were added 200 ml of petroleum ether and 200 ml of a 1 wt% aqueous solution of sodium chloride and agitated for one hour. The resulting solution was allowed to stand for sampling 316 ml of a petroleum ether phase as an upper phase. The resulting petroleum ether phase was further liquid-liquid extracted thrice with 200 ml of a 1 wt% aqueous solution of sodium chloride. The resulting solution was allowed to stand at 4°C for 12 hours and the resulting precipitate was recovered by filtration. As a result, 56 mg of dark purplish powders were obtained. The purity of the xanthophyll compound in the powders, as confirmed by high performance liquid chromatography, was found to be 99%.

### Example 2-11

To 200 ml of the THF-extracted colored liquid (B) prepared in the Preparation Example 2, were added 200 ml of cyclohexane and 200 ml of a 1 wt% aqueous solution of sodium hydroxide and agitated for one hour. The resulting solution was allowed to stand for sampling 305 ml of a cyclohexane phase as an upper phase. The resulting cyclohexane phase was further liquid-liquid extracted thrice with 200 ml of a 1 wt% aqueous solution of sodium chloride. The resulting solution was allowed to stand at 4°C for 12 hours and the resulting precipitate was recovered by filtration. As a result, 60 mg of dark purplish powders were obtained. The purity of the xanthophyll compound in the powders, as confirmed by high performance liquid chromatography, was found to be 98%.

### Example 2-12

To 200 ml of the THF-extracted colored liquid (B) prepared in the Preparation Example 2, were added 200 ml of hexane and 100 ml of a 1 wt% aqueous solution of sodium chloride and agitated for one hour. The resulting solution was allowed to stand for sampling 325 ml of a hexane phase as an upper phase. The resulting hexane phase was further liquid-liquid extracted thrice with 100 ml of a 1 wt% aqueous solution of sodium chloride. The resulting solution was allowed to stand at 4°C for 12 hours and the resulting precipitate was recovered by filtration. As a result, 53 mg of dark purplish powders were obtained. The purity of the xanthophyll compound in the powders, as confirmed by high performance liquid chromatography, was found to be 99%.

### Example 3-1

To 200 ml of the acetone-extracted colored liquid (A) prepared in the Preparation Example 1, were added 200 ml of hexane and 200 ml of a 1 wt% aqueous solution of sodium chloride and agitated for one hour. The resulting solution was allowed to stand for sampling 223 ml of a hexane phase as an upper phase. To the resulting hexane phase were added 400 ml of hexane. The resulting solution was allowed to stand at 4°C for 12 hours and the resulting precipitate was recovered by filtration. As a result, 11.0 mg of dark purplish powders were obtained. The purity of the xanthophyll compound in the powders, as confirmed by high performance liquid chromatography, was found to be 98%.

### Example 3-2

To 200 ml of the acetone-extracted colored liquid (A) prepared in the Preparation Example 1, were added 200 ml of hexane and 200 ml of a 1 wt% aqueous solution of sodium chloride and agitated for one hour. The resulting solution was allowed to stand for sampling 223 ml of a hexane phase as an upper phase. To the resulting hexane phase were added 400 ml of petroleum ether. The resulting solution was allowed to stand at 4°C for 12 hours and the resulting precipitate was recovered by filtration. As a result, 11.1 mg of dark purplish powders were obtained. The purity of the xanthophyll compound in the powders, as confirmed by high performance liquid chromatography, was found to be 98%.

### Example 3-3

To 200 ml of the acetone-extracted colored liquid (A) prepared in the Preparation Example 1, were added 200 ml of hexane and 200 ml of city water and agitated for one hour. The resulting solution was allowed to stand for sampling 218 ml of a hexane phase as an upper phase. To the resulting hexane phase were added 400 ml of hexane. The resulting solution was allowed to stand at 4°C for 12 hours and the resulting precipitate was recovered by filtration. As a result, 11.6 mg of dark purplish powders were obtained. The purity of the xanthophyll compound in the powders, as confirmed by high performance liquid chromatography, was found to be 99%.

### Example 3-4

To 200 ml of the acetone-extracted colored liquid (A) prepared in the Preparation Example 1, were added 200 ml of petroleum ether and 200 ml of a 1 wt% aqueous solution of sodium chloride and agitated for one hour. The resulting solution was allowed to stand for sampling 221 ml of a petroleum ether phase as an upper phase. To the resulting petroleum ether phase were added 400 ml of petroleum ether. The resulting solution was allowed to stand at 4°C for 12 hours and the resulting precipitate was recovered by filtration. As a result, 11.3 mg of dark purplish powders were obtained. The purity of the xanthophyll compound in the powders, as confirmed by high performance liquid chromatography, was found to be 99%.

### Example 3-5

To 200 ml of the acetone-extracted colored liquid (A) prepared in the Preparation Example 1, were added 200 ml of cyclohexane and 200 ml of a 1 wt% aqueous solution of sodium chloride and agitated for one hour. The resulting solution was allowed to stand for sampling 226 ml of the cyclohexane phase as an upper phase. To the resulting cyclohexane phase were added 400 ml of cyclohexane. The resulting solution was allowed to stand at 4°C for 12 hours and the resulting precipitate was recovered by filtration. As a result, 12.0 mg of dark purplish powders were obtained. The purity of the xanthophyll compound in the powders, as confirmed by high performance liquid chromatography, was found to be 99%.

### Example 3-6

To 200 ml of the acetone-extracted colored liquid (A) prepared in the Preparation Example 1, were added 200 ml of hexane and 100 ml of a 1 wt% aqueous solution of sodium chloride and agitated for one hour. The resulting solution was allowed to stand for sampling 235 ml of a hexane phase as an upper phase. To the resulting hexane phase were added 600 ml of hexane. The resulting solution was allowed to stand at 4°C for 12 hours and the resulting precipitate was recovered by filtration. As a result, 11.5 mg of dark purplish powders were obtained. The purity of the xanthophyll compound in the powders, as confirmed by high performance liquid chromatography, was found to be 98%.

### Example 3-7

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. To the resulting hexane phase were added 800 ml of hexane. The resulting solution was allowed to stand at 4°C for 12 hours and the resulting precipitate was recovered by filtration. As a result, 53 mg of dark purplish powders were obtained. The purity of the xanthophyll compound in the powders, as confirmed by high performance liquid chromatography, was found to be 98%.

### Example 3-8

To 200 ml of the THF-extracted colored liquid (B) prepared in the Preparation Example 2, were added 200 ml of hexane and 200 ml of a 1 wt% aqueous solution of sodium chloride and agitated for one hour. The resulting solution was allowed to stand for sampling 312 ml of a hexane phase as an upper phase. To the resulting hexane phase were added 800 ml of petroleum ether. The resulting solution was allowed to stand at 4°C for 12 hours and the resulting precipitate was recovered by filtration. As a result, 54 mg of dark purplish powders were obtained. The purity of the xanthophyll compound in the powders, as confirmed by high performance liquid chromatography, was found to be 98%.

### Example 3-9

To 200 ml of the THF-extracted colored liquid (B) prepared in the Preparation Example 2, were added 200 ml of hexane and 200 ml of city water and agitated for one hour. The resulting solution was allowed to stand for sampling 301 ml of a hexane phase as an upper phase. To the resulting hexane phase were added 800 ml of hexane. The resulting solution was allowed to stand at 4°C for 12 hours and the resulting precipitate was recovered by filtration. As a result, 56 mg of dark purplish powders were obtained. The purity of the xanthophyll compound in the powders, as confirmed by high performance liquid chromatography, was found to be 97%.

### Example 3-10

To 200 ml of the THF-extracted colored liquid (B) prepared in the Preparation Example 2, were added 200 ml of petroleum ether and 200 ml of a 1 wt% aqueous solution of sodium chloride and agitated for one hour. The resulting solution was allowed to stand for sampling 316 ml of a petroleum ether phase as an upper phase. To the resulting petroleum ether phase were added 800 ml of petroleum ether. The resulting solution was allowed to stand at 4°C for 12 hours and the resulting precipitate was recovered by filtration. As a result, 57 mg of dark purplish powders were obtained. The purity of the xanthophyll compound in the powders, as confirmed by high performance liquid chromatography, was found to be 99%.

### Example 3-11

To 200 ml of the THF-extracted colored liquid (B) prepared in the Preparation Example 2, were added 200 ml of cyclohexane and 200 ml of a 1 wt% aqueous solution of sodium chloride and agitated for one hour. The resulting solution was allowed to stand for sampling 305 ml of a cyclohexane phase as an upper phase. To the resulting cyclohexane phase were added 800 ml of cyclohexane. The resulting solution was allowed to stand at 4°C for 12 hours and the resulting precipitate was recovered by filtration. As a result, 59 mg of dark purplish powders were obtained. The purity of a xanthophyll compound in the powders, as confirmed by high performance liquid chromatography, was found to be 100%.

### Example 3-12

To 200 ml of the THF-extracted colored liquid (B) prepared in the Preparation Example 2, were added 200 ml of hexane and 100 ml of a 1 wt% aqueous solution of sodium chloride and agitated for one hour. The resulting solution was allowed to stand for sampling 325 ml of a hexane phase as an upper phase. To the resulting hexane phase were added 1200 ml of hexane. The resulting solution was allowed to stand at 4°C for 12 hours and the resulting precipitate was recovered by filtration. As a result, 61 mg of dark purplish powders were obtained. The purity of the xanthophyll compound in the powders, as confirmed by high performance liquid chromatography, was found to be 99%.

It is seen from the above Examples that any of the step (c-1) of concentrating the nonpolar solvent phase, the step (c-2) of adding water to the nonpolar solvent phase followed by further subjecting at least once to liquid-liquid extraction and the step (c-3) of further adding a nonpolar solvent to the nonpolar solvent phase is effective in purifying the xanthophyll compound and that any suitable combination of these steps (c-1) to (c-3) also gives high purity xanthophyll compounds.

## Claims

1. A method for purifying a xanthophyll compound comprising the steps of:
(a) adding a nonpolar solvent and water to an aqueous organic solvent solution containing the xanthophyll compound;
(b) subjecting to liquid-liquid extraction to thereby obtain a nonpolar solvent phase; and
(c) subjecting the nonpolar solvent phase to at least one step selected from the following steps (c-1) to (c-3):
step (c-1) of concentrating the nonpolar solvent phase;
step (c-2) of adding water to the nonpolar solvent phase followed by further subjecting at least once to liquid-liquid extraction; and
step (c-3) of further adding a nonpolar solvent to the nonpolar solvent phase
whereby said xanthophyll compound is precipitated.

2. The method as claimed in claim 1 wherein said non-polar solvent in said step (a) is selected from hexane, heptane, octane, cyclohexane, benzene, toluene, xylene, petroleum ether, naphtha, kerosene, n-paraffin and mixtures thereof.

3. The method as claimed in claim 1 wherein said water in said step (a) is selected from distilled water, ion-exchanged water, city water, industrial water, an aqueous solution of sodium hydroxide, an aqueous solution of sodium chloride, sea water and mixtures thereof.

4. The method as claimed in claim 1 wherein 0.1 to 20 parts by volume of the nonpolar solvent and not less than 0.1 part by volume of said water are added in said step (a) to 1 part by volume of said aqueous organic solvent solution containing the xanthophyll compound.

5. The method as claimed in claim 1 wherein said water in said step (c-2) is selected from distilled water, ion-exchanged water, city water, industrial water, an aqueous solution of sodium hydroxide, an aqueous solution of sodium chloride, sea water and mixtures thereof.

6. The method as claimed in claim 1 wherein not less than 0.1 part by volume of said water is added in said step (c-2) to 1 part by volume of said nonpolar solvent phase.

7. The method as claimed in claim 1 wherein said non-polar solvent in said step (c-3) is selected from hexane, heptane, octane, cyclohexane, benzene, toluene, xylene, petroleum ether, naphtha, kerosene, n-paraffin and mixtures thereof.

8. The method as claimed in claim 1 wherein not less than 0.1 part by volume of said nonpolar solvent is added in said step (c-3) to 1 part by volume of said nonpolar solvent phase.

9. The method as claimed in claim 1 wherein said xanthophyll compound is selected from astaxanthin, canthaxanthin, zeaxanthin, adonixanthin, adonirubin, β-cryptoxanthin, echinenone, asteroidenone, 3-hydroxyechinenone, rhodoxanthin, fucoxanthin, lutein, capsanthin, capsorubin and mixtures thereof.
